# EUROPEAN PATENT SPECIFICATION

(11) **EP 3 721 790 B1**
(45) Date of publication and mention of the grant of the patent: **25.10.2023**
(21) Application number: 19754360.6
(22) Date of filing: 24.01.2019
(51) Int. Cl.: A61B 5/00, G16H 40/40, A61B 5/145, A61B 5/1486

(54) **CONTINUOUS BIOMETRIC INFORMATION MEASURING DEVICE USING SENSOR USAGE INFORMATION STORED IN MEMORY**
VORRICHTUNG ZUR KONTINUIERLICHEN MESSUNG BIOMETRISCHER INFORMATIONEN UNTER VERWENDUNG VON IN EINEM SPEICHER GESPEICHERTEN SENSORNUTZUNGSINFORMATIONEN
DISPOSITIF DE MESURE D'INFORMATIONS BIOMÉTRIQUES CONTINUE FAISANT APPEL À DES INFORMATIONS D'UTILISATION DE CAPTEUR MÉMORISÉES DANS UNE MÉMOIRE

(30) Priority: 14.02.2018 KR 20180018400
(43) Date of publication of application: 14.10.2020
(73) Proprietor: i-Sens, Inc., Seoul 06646 (KR)
(72) Inventor: PARK, Jeong Je, Hwaseong-si Gyeonggi-do 18442 (KR); LEE, Jin Won, Seoul 02598 (KR); NAH, Ji Seon, Seoul 07044 (KR); PARK, Hyo Seon, Seoul 07287 (KR); KIM, Jin Ho, Seoul 08786 (KR); LEE, Ha Na, Seoul 02145 (KR)
(74) Representative: Sander, Rolf
(86) International application number: PCT/KR2019/001011
(87) International publication number: WO 2019/160255

(56) References cited:
- EP-A1- 2 332 466
- KR-A- 20090 079 940
- KR-A- 20110 004 845
- KR-A- 20130 024 468
- KR-A- 20150 067 047
- KR-A- 20170 008 216
- US-A1- 2010 274 515
- US-A1- 2014 024 908
- US-A1- 2016 232 322

## Description

### TECHNICAL FIELD

The present disclosure relates to a continuous biometric information measuring device and, more specifically, to a continuous biometric information measuring device having a separate memory for storing sensor usage information in a sensor module to help a user effectively use the continuous biometric information measuring device or prevent reuse or shared use of the continuous biometric information measuring device, using the sensor usage information, or determine a remaining use period of the sensor module on the basis of the sensor usage information stored in the sensor module, thereby informing a user of the need for replacement of the sensor module depending on the remaining use period.

### BACKGROUND ART

Diabetes is a chronic disease common in modern people, and about 400 million people in the world suffer from diabetes.

Diabetes is caused by the pancreas producing an entirely, or relatively, insufficient amount of insulin due to a variety of reasons, such as obesity, stress, and bad eating habits, and due to congenital or hereditary reasons, so that glucose levels are absolutely high in blood, instead of being balanced in blood.

Blood contains a certain concentration of glucose, from which tissue cells produce energy.

However, when glucose levels are higher than normal, glucose is not stored appropriately in the liver, muscles, or fat cells and excessive amounts of glucose remain in the blood. Thus, diabetic patients commonly have higher blood glucose levels than other people. Excessive amounts of blood glucose may be discharged in urine without being absorbed by body tissues. Accordingly, since the body tissues may fail to have sufficient amounts of glucose, necessary for normal functioning, the body tissues may malfunction.

Diabetes has subtle or no subjective symptoms in the early stages of the disease. With the progression of the disease, however, the classic symptoms of diabetes, such as polydipsia, polyphagia, polyuria, weight loss, fatigue, itchy skin, and slow healing of cuts of the hands and feet, appear. Prolonged diabetes may cause complications, such as blurred vision, high blood pressure, nephropathy, palsy, periodontal diseases, muscle spasms, neuralgia, and gangrene.

In order to diagnose and manage diabetes such that complications do not arise, systematic measurement of blood glucose should be carried out in concert with systematic treatment.

A glucose monitoring device uses a method in which a user performs a blood glucose measurement once by drawing blood from a fingertip and a method in which the glucose monitoring system is attached to a user's abdomen and arm and blood glucose measurement is continuously performed.

In the case of the one-by-one blood glucose measurement method using a disposable blood glucose strip sensor, since a blood sample should be taken by directly using a needle or the like every time a blood sample is to be obtained, there is a disadvantage in that pain or stress is accompanied in taking a blood sample. Thus, a continuous blood glucose measurement technology using a continuous blood glucose sensor has recently been developed.

Moreover, in the case of a diabetic patient having progressed into a serious condition, a blood glucose level needs to be more strictly managed, so a continuous blood glucose measuring device that continuously performs blood glucose measurement is used.

The continuous blood glucose meter includes a sensor module attached to the user's skin to periodically measure the user's blood glucose from a user's body fluid, a transmitter that transmits the blood glucose information measured by the sensor module to a user terminal, and the like. The sensor module is provided with a sensor probe for extracting a body fluid and a sensor for measuring blood glucose from the extracted body fluid.

Typically, the sensor module of the continuous blood glucose meter has a certain expiration date according to the measurement method, such as enzymatic method/non-enzymatic method, so the user needs to measure the user's blood glucose information by attaching a sensor module that has not past the expiration date to the skin. However, there may be cases in which a user accidentally uses a sensor module whose expiration date has elapsed, or a user intentionally uses a sensor module whose expiration date has elapsed since he/she imagines the sensor module to still be useful.

In such cases in which a user accidentally uses a sensor module whose expiration date has elapsed, or a user intentionally uses a sensor module whose expiration date has elapsed since he/she imagines the sensor module to still be useful, by using the sensor module of the continuous blood glucose meter whose expiration date has elapsed, the user may obtain inaccurate measurements that are beyond the accuracy performance guaranteed by the manufacturer, so that the user may not receive proper blood glucose control and treatment due to inaccurate blood glucose information.

Moreover, if reused or shared by multiple users, the sensor module of the continuous blood glucose measuring device may cause significant health problems, such as infections, due to the nature of the sensor module of being inserted into the body. Thus, the sensor module should only be used once, and should not be shared by multiple users. However, the sensor module of the conventional continuous blood glucose measuring device may have a problem, in that there may be no measure for solving the problem of reuse or shared use.
US2014024908 (HAGI KOUJI [JP]) discloses a body fluid component measuring system which is capable of continuous measurement and reduces downtime includes a sensor unit which is positionable indwelling in the skin of a subject and which measures a predetermined body fluid component included in the body fluid of the subject; and a transmission unit which is detachably attached to the sensor unit and which calculates blood glucose level based on measurement data measured by the sensor unit, and transmits the calculated blood glucose level to a display unit.
EP2332466 (DEXCOM INC [US]) discloses a transcutaneous analyte sensor system can include an applicator, a mounting unit, an electronics unit, a base adapted for mounting on the skin of a host, and one or more contacts configured to provide secure electrical contact between the sensor and the electronics unit.

### DISCLOSURE

### Technical Problem

Accordingly, the present disclosure has been made in consideration of the above-described problems occurring in the related art, and an objective of the present disclosure is to provide a continuous biometric information measuring device having a separate memory for storing sensor usage information in a sensor module to help a user effectively use the continuous biometric information measuring device or prevent reuse or shared use of the continuous biometric information measuring device.

Another objective of the present disclosure is to provide a continuous biometric information measuring device capable of determining the remaining use period of a sensor module on the basis of sensor usage information stored in a memory of the sensor module to inform the user of the replacement of the sensor module according to the remaining use period.

A further objective of the present disclosure is to provide a continuous biometric information measuring device in which an identifier of a transmitter connected to a sensor module is stored in a memory so that in the event of replacing only the transmitter other than the sensor module, user's biometric information is continuously measured through the sensor module along with omitting an initialization of the sensor module including a stabilization or calibration.

### Technical Solution

In accordance with an aspect of the present invention, there is provided a continuous biometric information measuring device as defined by claim 1. Further advantageous embodiments of the present invention are defined by the dependent claims.

According to an aspect of the present disclosure, a continuous biometric information measuring device includes: a sensor module positioned on a user's skin and periodically measure user's biological information from the body fluid; and a transmitter connected to the sensor module in a detachable manner to transmit the biometric information measured by the sensor module to a user terminal, wherein the sensor module includes a separate memory storing sensor usage information therein. The sensor module is further configured to receive a transmitter identifier from the transmitter upon connection of the transmitter to the sensor module and store the transmitted identifier in the memory; determine whether the transmitter is a replaced transmitter; in response to determining that the transmitter is a replaced transmitter, compare the stored transmitter identifier with a previously stored transmitter identifier to determine whether the replaced transmitter is a new transmitter; and if the replaced transmitter is determined to be a new transmitter, continuously measure biometric information without performing an initialization operation of the sensor module.

Here, the sensor usage information may include registered sensor usage information previously registered in the memory, generated sensor usage information generated when the sensor module and the transmitter are connected, and received sensor usage information received from the user terminal through the transmitter.

Here, the registered sensor usage information may be at least one of an identifier, a manufacturing date, and a production lot of the sensor module, the generated sensor usage information may be at least one of a use-start time of the sensor module and an identifier of the transmitter, and the receiving sensor usage information may be user information received from the user terminal.

Preferably, the sensor module may further include a storage controller generating the generated sensor usage information, registering and storing the generated sensor usage information in the memory, receiving the received sensor usage information, and registering and storing the received sensor usage information in the memory.

Here, the storage controller may generate a connection time or an initial pairing time as information on the use-start time by determining the connection time when the sensor module and the transmitter are connected or determining a time when an initial pairing connection is made between the transmitter and the user terminal and register and store the generated use-start information in the memory.

Preferably, the sensor module may further include an operation controller determining whether a new transmitter is connected to the sensor module on the basis of the registered and stored transmitter identifier, and if the new transmitter has been connected to the sensor module, continuously measuring the biometric information without performing an initialization operation of the sensor module.

Preferably, the sensor module may further include a message manager generating a measurement message from a combination of the measured biometric information and the registered and stored user information and transmitting the measurement message to the user terminal through the transmitter.

Here, the message manager may generate the measurement message by encrypting the measured biometric information with the user information.

Here, when generating the use-start time information, the storage controller may transmit the registered and stored manufacturing date information or production lot information of the sensor module to the user terminal through the transmitter. The manufacturing date information may be used by the user terminal to determine the expiration period of the sensor module, or the production lot information may be used by the user terminal to determine whether the sensor module is defective.

Here, the message manager may determine the usage period of the sensor module from the use-start time information and, when the usage period of the sensor module is determined to be expired, generate an expiration message, and transmit the expiration message to the user terminal through the transmitter.

### Advantageous Effects

The continuous biometric information measuring device according to the present disclosure has the following effects.

First, the continuous biometric information measuring device according to the present disclosure may include a separate memory for storing sensor usage information in a sensor module to help a user effectively use the continuous biometric information measuring device or prevent reuse or shared use of the continuous biometric information measuring device.

Second, the continuous biometric information measuring device according to the present disclosure may determine the remaining use period of a sensor module on the basis of sensor usage information stored in a memory of the sensor module to inform the user of the replacement of the sensor module according to the remaining use period.

Third, the continuous biometric information measuring device according to the present disclosure may store an identifier of a transmitter initially connected to a sensor module in a memory, so that in an event in which only the transmitter other than the sensor module is replaced with a new transmitter, user's biometric information may be continuously measured using the sensor module while the initialization of the sensor module including a stabilization or calibration is omitted.

### DESCRIPTION OF DRAWINGS

FIG. 1 is a diagram illustrating a continuous biometric information management system according to the present disclosure;
FIG. 2 is a diagram illustrating an example of a continuous biometric information measuring device 100 according to the present disclosure;
FIG. 3 is a functional block diagram illustrating a continuous biometric information measuring device according to the present disclosure;
FIG. 4 illustrates an example of sensor usage information stored in a storage; and
FIG. 5 illustrates an example of a warning message and an expiration-notification message according to the present disclosure.

### MODE FOR INVENTION

The technical or scientific terms used herein are for the purpose of describing particular exemplary embodiments only and are not intended to be limiting of the present disclosure. In addition, the technical or scientific terms used herein should be understood as having the same meaning as commonly understood by a person having ordinary knowledge in the art to which the present disclosure relates, and should not be interpreted in an overly broad or narrow manner, unless expressly so defined herein. If any technical terms used herein fail in correctly representing the idea of the present disclosure, they should be substituted with technical terms correctly understandable by a person having ordinary knowledge in the art to which the present disclosure relates.

Descriptions of components in the singular form used herein are intended to include descriptions of components in the plural form, unless explicitly described to the contrary. It will be understood that the terms "comprise," "include," and any variations thereof used herein shall not be interpreted as essentially including all components or steps described herein, and are intended to cover non-exclusive inclusions unless explicitly described to the contrary.

In addition, the accompanying drawings shall be interpreted as being provided for a better understanding, while not being limitative, of the principle of the present disclosure.

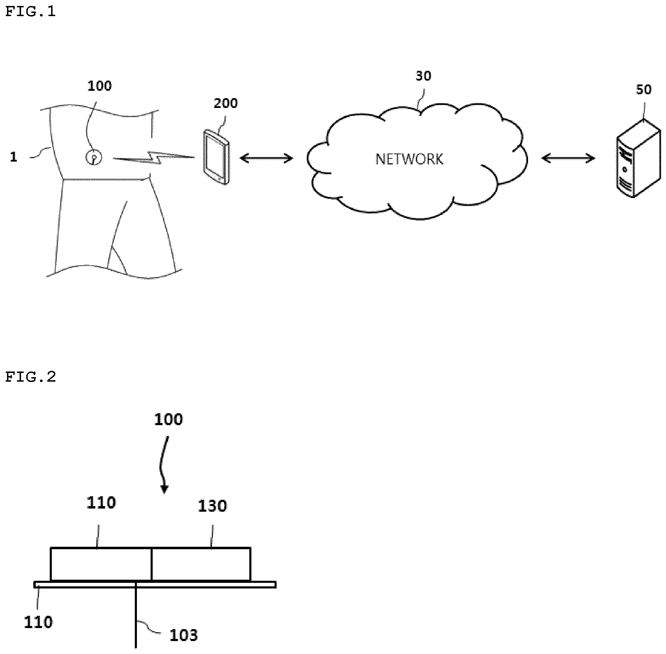

FIG. 1 is a diagram illustrating a continuous biometric information management system according to the present disclosure.

Referring specifically to FIG. 1, a continuous biometric information measuring device 100 is attached to the skin of the user 1. The continuous biometric information measuring device 100 periodically extracts the body fluid of the user 1 to measure the user's biometric information, for example, blood glucose, using the extracted body fluid.

A user terminal 10 is connected to the continuous biometric information measuring device 100 via short-range communication, for example, Bluetooth^{®} communication, infrared communication, and the like. The continuous biometric information measuring device 100 and the user terminal 10 transmit and receive data to and from each other via short-range communication. That is, when the continuous biometric information measuring device 100 and the user terminal 10 are located in a short-range communication range of each other, the continuous biometric information measuring device 100 transmits measured biometric information of the user 1 to the user terminal 10.

Here, the user terminal 10 is a device capable of storing, or outputting to the user, the user's biometric information received from the continuous biometric information measuring device 100 so that the user may review the information. The user terminal 10 may execute an application for managing the biometric information. As an example of such a user terminal 10, a smartphone, a notebook PC, a PDA, a dedicated terminal for managing biometric information, and the like may be used.

A management server 50 is connected to the user terminal 10 via the network 30. The user terminal 10 transmits the biometric information of the user 1 received from the continuous biometric information measuring device 100 to the management server 50 or the sensor usage information of the continuous biometric information measuring device 100 to the management server 50. Meanwhile, the user terminal 10 may receive product information for each production lot of the continuous biometric information measuring device from the management server 50.

Preferably, the user terminal 10 identifies a user on the basis of the user information, and registers and stores the received biometric information by mapping the received biometric information to the identified user. Meanwhile, the user terminal 10 transmits the registered biometric information mapped to the user or sensor usage information to the management server 50, together with the user information.

The continuous biometric information measuring device according to the present disclosure includes a sensor module that extracts a user's body fluid to measure user's biometric information, and a transmitter that transmits the measured biometric information to the user terminal. The sensor module includes a separate memory storing sensor usage information to allow the user to use an effective continuous biometric information measuring device by using the sensor usage information, or to prevent reuse, or shared use by multiple users, of the continuous biometric information measuring device.

FIG. 2 is a view illustrating an example of a continuous biometric information measuring device 100 according to the present disclosure.

The continuous biometric information measuring device 100 according to the present disclosure includes a sensor module 110 attached to the user's skin to extract the user's body fluid and periodically measure the user's biometric information from the body fluid, and a transmitter 130 detachably connected to the sensor module 110 to transmit the biometric information measured by the sensor module 110 to the user terminal.

A connection terminal (not shown) is formed on one side of the sensor module 110 so that the sensor module 110 and the transmitter 130 are connected to each other through the connection terminal. The biometric information measured by the sensor module 110 may be transmitted to the transmitter 130 through the connection terminal.

The sensor module 110 and the transmitter 130 are disposed on the user's skin through an attachment pad 101, formed to be attached to a part of the body, for example, the skin of the belly or flank. The attachment methods of the attachment pad 101 to the body may be set in various manners, such as an attachment method using a separate fixing band (not shown) or an attachment method in which an adhesive is applied to the edge portion so that the adhesive edge portion is attached to the skin.

At this time, a body fluid extraction tube 103 may be arranged to be partially inserted into the body while the attachment pad 101 is attached to the body. For example, the body fluid extraction tube is disposed such that an end thereof protrudes from a body's attachment surface of the attachment pad 101. According to this structure, when the attachment pad 101 is attached to the body surface, the body fluid extraction tube 103 attached to the attachment pad 101 is disposed such that the end portion thereof is inserted into the body.

Here, the body fluid may include either cell interstitial fluid or blood, and the blood conceptually includes any one of capillary blood, venous blood, and arterial blood. In addition, the body fluid extraction tube 103 may be provided in various forms capable of extracting body fluids from the body. Such forms including cannula, microdialysis tubing, or the like are collectively referred to as a body fluid extraction tube.

When the attachment pad 101 is attached to the body in this manner, since the body fluid extraction tube 103 is maintained in the body, the user's body fluid, extracted through the body fluid extraction tube 103 in this state and fed through a connection tube, is measured with respect to biometric information, for example, blood glucose concentration, by the sensor module 110.

The sensor module 110 measures the blood glucose concentration of the body fluid using various types of sensors, such as a measuring sensor used in a conventional blood collection-type blood glucose meter. In addition, since existing various blood glucose sensors may be applied, a detailed description thereof will be omitted.

The user's biometric information measured by the sensor module 110 is transmitted through the transmitter 130 to a connected user terminal (when a short-range communication is Bluetooth communication, a paired user terminal) located in a short-range communication range.

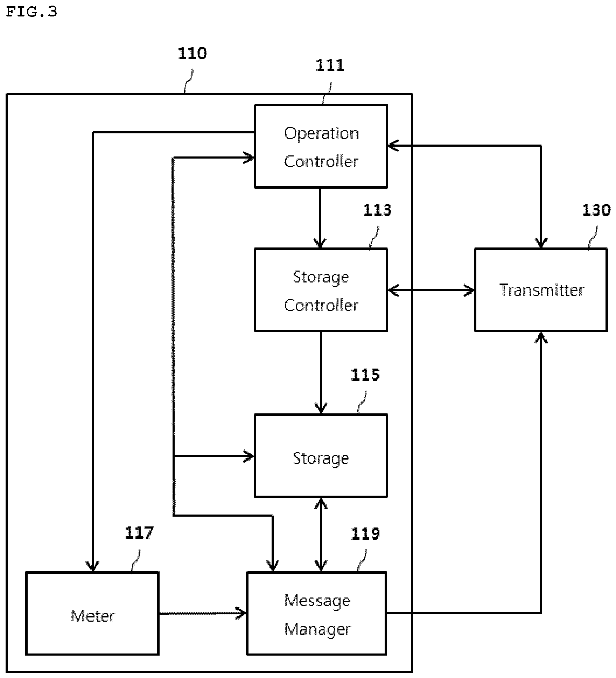

FIG. 3 is a functional block diagram illustrating a continuous biometric information measuring device according to the present disclosure.

Referring more specifically to FIG. 3, when the sensor module 110 and the transmitter 130 are connected to each other through a connection terminal, an operation controller 111 determines whether the sensor module and the transmitter are connected. Here, the sensor module 110 and the transmitter 130 are manufactured as separate modules, and the sensor module 110 and the transmitter 130 are fastened in a detachable manner.

Meanwhile, the operation controller 111 initializes a meter (or measuring element) 117 by determining whether the sensor module 110 touches the user's skin. The meter 117 performs an initialization operation, such as a stabilization stage and a calibration stage, to start measuring biometric information under the control of the operation controller 111. That is, since the meter 117 is coated with a thin film of glucose oxidase and inserted into the subcutaneous tissue, the meter performs the initialization operation, for example, by performing the stabilization stage of about 2 hours before the coated glucose oxidase is hydrated with an aqueous solution so that the biometric information may be precisely measured, or Self-Monitoring of Blood Glucose (SMBG) calibration stage.

Here, the operation controller 111 may determine whether the sensor module 110 is in contact with the user's skin by determining whether the sensor module and the transmitter 130 are connected, whether the pairing between the sensor module and a user terminal through the transmitter 130 is completed, or whether extracted body fluid is detected by the meter 117.

The sensor module 110 according to the present disclosure is provided with a separate storage 115 and a storage controller 113 for managing information stored in the storage 115.

The storage 115 registers and stores sensor usage information to manage the effective use of the sensor module 110, prevents reuse or shared use of the sensor module, or informs the user of the need for replacement of the sensor module. The sensor usage information registered and stored in the storage 115 includes sensor usage information previously registered in the storage 115 at shipping time, sensor usage information generated when the sensor module 110 is connected to the transmitter, and sensor usage information received from the user terminal via the transmitter 130.

Here, the registered sensor usage information is an identifier of the sensor module, a manufacturing date, a production lot, an expiration date, a usage period or the number of uses, and the generated sensor usage information is a start time when the sensor module is used, and an identifier of a connected transmitter, and the received sensor usage information is the user information received from the user terminal.

The production lot information is provided for the following reason. Since the meter of the sensor module of the blood glucose meter is a complex of several substances, such as a metal, an enzyme, a film, or the like, and the production conditions thereof, such as temperature, humidity, and user's proficiency, are also different, it is theoretically impossible to produce sensor modules with the same performance and properties. Thus, mass-produced sensor modules may be divided into lots based on production lot information so that the deviations of the sensor modules that appear according to the production lot information may be determined, thereby accurately measuring biometric information.

Here, the user information may be information of a biometric information management application executed in a user terminal. For example, the information may include a user ID or a password.

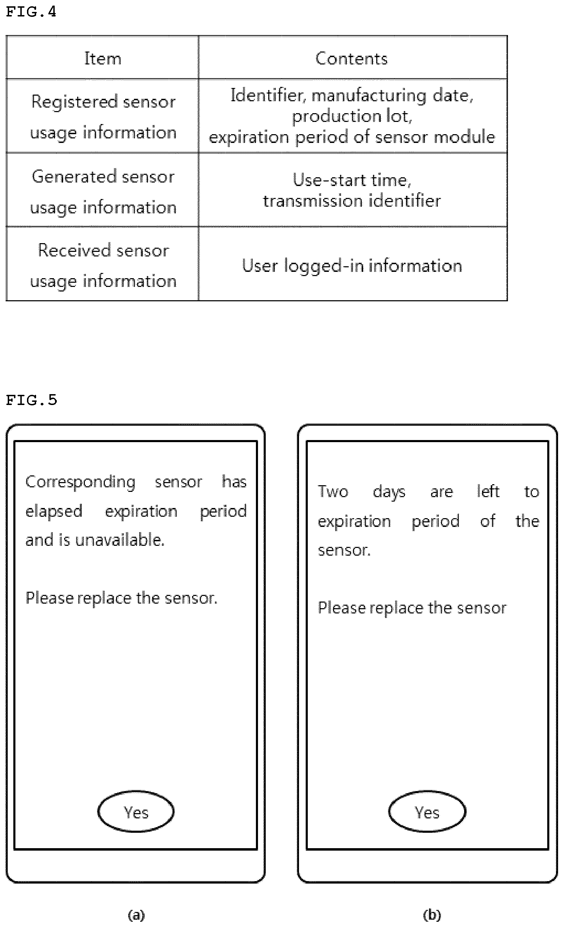

FIG. 4 illustrates an example of sensor usage information stored in the storage 115 of the sensor module. According to the field to which the present disclosure is applied, various different types of sensor usage information may be stored in the storage 115, which falls within the scope of the present disclosure.

Referring back to FIG. 3, the storage controller 113 manages information stored in the storage 115, and the storage controller 113 receives information about the status of the connection or the transmitter identifier from the operation controller 111 when the sensor module 110 and the transmitter 130 are connected.

The storage controller 113 determines the connection time at which the sensor module and the transmitter are connected or receives the initial pairing time between the user terminal and the continuous biometric information measuring device from the transmitter. The storage controller 113 generates the connection time or the initial pairing time as use-start time information of the continuous biometric information measuring device and stores and registers the generated use-start time information in the storage 115.

In addition, the storage controller 113 receives user information from the user terminal paired through the transmitter 130 and stores and registers the received information in the storage 115.

Meanwhile, when the meter 117 completes the initialization operation, the operation controller 111 controls the meter 117 to periodically extract the user's body fluid and measure the user's biometric information. The meter 117 provides the periodically measured user's biometric information to a message manager 119. The message manager 119 generates a measurement message by combining the biometric information and sensor usage information registered and stored in the storage 115.

Here, the measurement message is generated from a combination of biometric information and user information, wherein the user information is user information stored in the storage 115. Preferably, the message manager 119 may generate a measurement message by encrypting biometric information with stored user information.

With the provision of stored user information in the measurement message or encryption of the measurement message with stored user information, when receiving the measurement message, the user terminal compares the user information in the measurement message with the user information input to the application of the user terminal to map the identical user and manage the biometric information thereof. This may prevent third parties from being able to view the user's biometric information, or prevent different users from sharing or reusing the same sensor module.

On the other hand, the continuous biometric information measuring device according to the present disclosure is applied with the configuration to prevent the use of the continuous biometric information measuring device that is not valid. More specifically, the operation controller 111 compares expiration period in the registered sensor usage information stored in the storage 115 and the connection time in the generated sensor usage information to determine whether or not an expiration period of the continuous biometric information measuring device has expired, or transmits the production lot information to the user terminal through the transmitter 130 to determine whether or not the continuous biometric information measuring device corresponding to the lot information is a defective product. The user terminal stores product information for each production lot of the continuous biometric information measuring device, received from the management server, that is, information about a defective product.

When the expiration period of the continuous biometric information measuring device has expired or when the continuous biometric information measuring device is defective, the operation controller 111 may control the meter 117 to interrupt the measurement of biometric information; control the transmitter to interrupt the transmission of measured biometric information to the user terminal; or control a message manager 119 to transmit a warning message.

Preferably, the operation controller 111 may generate and transmit a warning message at the earliest time detecting the user's usage of the sensor module 110 to induce the replacement of the sensor module before the user inserts the body fluid extraction tube of the sensor module 110 into the user's skin. For example, when detecting the connection between the sensor module 110 and the transmitter 130, the operation controller 111 may first determine the expiration period of the sensor module 110 or determine whether the sensor module 110 is defective before controlling a warning message to be transmitted.

FIG. 5 (a) illustrates a warning message according to an embodiment of the present disclosure, wherein if an expired sensor module is to be used, on the basis of the expiration period stored in the storage 115 and use-start time information, the message manager 119 may transmit a warning message to the user terminal, the warning message indicating that the corresponding sensor module should be replaced with a new sensor module without using the corresponding sensor module.

Referring back to FIG. 3, the message manager 119 may calculate the remaining use period of the sensor module on the basis of the use-start time information stored in the storage 115, the usage period of the corresponding sensor module, and the current date information, and generate an expiration guide message on the basis of the remaining use period and transmit the generated expiration guide message to the user terminal through the transmitter 130. Here, the expiration guide message may include information on the remaining use period or a warning phrase indicating that the sensor module should not to be used when the remaining use period has expired.

For example, when the remaining use period calculated on the basis of the use-start time information, the usage period of the corresponding sensor module, and the current date information is less than a threshold date, the message manager 119 may generate an expiration guide message and transmit the same to the user terminal.

As another example, when the remaining use period calculated on the basis of the use-start time information, the usage period of the corresponding sensor module, and the current date information has completely expired, the message manager 119 may generate an expiration guide message and transmit the same to the user terminal.

FIG. 5 (b) illustrates an example of an expiration guide message according to the present disclosure. A control operation is performed so that an expiration guide message including the remaining use period of the corresponding sensor module, as illustrated in FIG. 5 (b), is generated and output to the user terminal for activation.

Referring back to FIG. 3, in the case of the continuous biometric information measuring device according to the present disclosure, the sensor module 110 and the transmitter 130 are manufactured as separate modules, respectively. Here, although the sensor module 110 is periodically used for each predetermined period of use, the transmitter 130 may be used continuously for a longer period of use than the sensor module 110. Therefore, after a predetermined period of use, the sensor module 110 is separated from the transmitter 130 and discarded, and a new sensor module is combined with the existing transmitter 130 to measure biometric information.

Whenever the new sensor module 110 is coupled to the transmitter 130, the operation controller 111 initializes the meter 117 of the sensor module, wherein the initialization operation usually takes 2 to 3 hours.

However, in some cases, in a situation in which remaining use period of the sensor module is left, the sensor module may be used as is, whereas the transmitter is replaced with a new transmitter, due to the failure of the transmitter or the power exhaustion. In such cases, when the new transmitter is coupled to the sensor module, the operation controller 111 determines whether the transmitter identifier stored in the storage 115 is the same as the identifier of the new transmitter. When the transmitter identifier stored in the storage 115 is different from the new transmitter identifier, the operation controller 111 determines that the sensor module should be used as is and only the transmitter should be replaced, and thus, controls the sensor module meter 117 to continuously measure the biometric information without initializing the meter 117.

As described above, the operation controller 111 may control the initialization operation of the sensor module depending on whether the sensor module or the transmitter is replaced, so that the initialization of the sensor module is not performed repeatedly. In this manner, the transmitter may be replaced without the initialization operation to immediately measure the user's biometric information.

While the present disclosure has been described with reference to certain exemplary embodiments shown in the drawings, these embodiments are illustrative only. Rather, it will be understood by a person having ordinary knowledge in the art that various modifications and equivalent other embodiments may be made therefrom. Therefore, the true scope of the present disclosure shall be defined by the concept of the appended claims.

## Claims

1. A continuous biometric information measuring device comprising:
a sensor module (110) positioned on a user's skin and periodically measure user's biological information from the body fluid, the sensor module (110) including a separate memory (115) for storing sensor usage information therein; and
a transmitter (130) connected to the sensor module (110) in a detachable manner to transmit the biometric information measured by the sensor module to a user terminal (10), **characterized in that:**
the sensor module (110) further configured to:receive a transmitter identifier from the transmitter (130) upon connection of the transmitter to the sensor module and store the transmitted identifier in the memory (115);
determine whether the transmitter is a replaced transmitter;
in response to determining that the transmitter is a replaced transmitter, compare the stored transmitter identifier with a previously stored transmitter identifier to determine whether the replaced transmitter is a new transmitter; and
if the replaced transmitter is determined to be a new transmitter, continuously measure biometric information without performing an initialization operation of the sensor module (110).

2. The continuous biometric information measuring device according to claim 1, wherein the sensor usage information includes registered sensor usage information previously registered in the memory (115), generated sensor usage information generated when the sensor module (110) and the transmitter (130) are connected, and received sensor usage information received from the user terminal (10) through the transmitter (130).

3. The continuous biometric information measuring device according to claim 2, wherein the registered sensor usage information is at least one of an identifier, a manufacturing date, and a production lot of the sensor module (110),
the generated sensor usage information is at least one of a use-start time of the sensor module (110) and an identifier of the transmitter (130), and
the receiving sensor usage information is user information received from the user terminal (10).

4. The continuous biometric information measuring device according to claim 3, wherein the sensor module (110) further includes a storage controller (113) configured to generate the generated sensor usage information, register and store the generated sensor usage information in the memory (115), receive the received sensor usage information, and register and store the received sensor usage information in the memory (115) .

5. The continuous biometric information measuring device according to claim 4, wherein the storage controller (113) configured to generate a connection time or an initial pairing time as information on the use-start time by determining the connection time when the sensor module (110) and the transmitter (130) are connected or determining a time when an initial pairing connection is made between the transmitter (130) and the user terminal (10) and register and store the generated use-start information in the memory (115).

6. The continuous biometric information measuring device according to claim 4, wherein the sensor module (110)further comprises an operation controller (111) configured to determine whether a new transmitter is connected to the sensor module on the basis of the registered and stored transmitter identifier, and if the new transmitter is connected to the sensor module, continuously measuring the biometric information without performing an initialization operation of the sensor module.

7. The continuous biometric information measuring device according to claim 4, wherein the sensor module (110) further comprises a message manager (119) configured to generate a measurement message from a combination of the measured biometric information and the registered and stored user information and transmit the measurement message to the user terminal (10) through the transmitter (130).

8. The continuous biometric information measuring device according to claim 7, wherein the message manager (119) configured to generate the measurement message by encrypting the measured biometric information with the user information.

9. The continuous biometric information measuring device according to claim 4, wherein, when generating the use-start time information, the storage controller (113) configured to transmit registered and stored manufacturing date information or production lot information of the sensor module (110) to the user terminal (10) through the transmitter (130),
wherein the manufacturing date information is used by the user terminal (10) to determine the expiration period of the sensor module (110), or the production lot information is used by the user terminal (10) to determine whether the sensor module (110) is defective.

10. The continuous biometric information measuring device according to claim 7, wherein the message manager (119) configured to determine the usage period of the sensor module (110) from the use-start time information and, when the usage period of the sensor module is determined to be expired, generate an expiration message, and transmits the expiration message to the user terminal (10) through the transmitter (130) .

## Patentansprüche

1. Vorrichtung zur kontinuierlichen Messung biometrischer Informationen, umfassend:
ein Sensormodul (110), das auf der Haut eines Benutzers positioniert ist und regelmäßig biologische Informationen des Benutzers aus der Körperflüssigkeit misst, wobei das Sensormodul (110) einen separaten Speicher (115) zum Speichern von Sensornutzungsinformationen enthält; und
einen Sender (130), der lösbar mit dem Sensormodul (110) verbunden ist, um die von dem Sensormodul gemessenen biometrischen Informationen an ein Benutzerendgerät (10) zu senden, **dadurch gekennzeichnet, dass:**
das Sensormodul (110) ferner zu Folgendem konfiguriert ist: Empfangen einer Senderkennung von dem Sender (130), wenn der Sender mit dem Sensormodul verbunden wird, und Speichern der übertragenen Kennung in dem Speicher (115);
Feststellen, ob der Sender ein Austauschsender ist,
als Reaktion auf ein Feststellen, dass der Sender ein Austauschsender ist, Vergleichen der gespeicherten Senderkennung mit einer zuvor gespeicherten Senderkennung, um festzustellen, ob der Austauschsender ein neuer Sender ist, und
wenn festgestellt wird, dass der Austauschsender ein neuer Sender ist, kontinuierliches Messen biometrischer Informationen, ohne einen Initialisierungsvorgang des Sensormoduls (110) durchzuführen.

2. Vorrichtung zur kontinuierlichen Messung biometrischer Informationen nach Anspruch 1, wobei die Sensornutzungsinformationen registrierte Sensornutzungsinformationen, die zuvor in dem Speicher (115) registriert wurden, generierte Sensornutzungsinformationen, die generiert wurden, als das Sensormodul (110) und der Sender (130) angeschlossen wurden, und empfangene Sensornutzungsinformationen, die von dem Benutzerendgerät (10) über den Sender (130) empfangen wurden, beinhalten.

3. Vorrichtung zur kontinuierlichen Messung biometrischer Informationen nach Anspruch 2, wobei die registrierten Sensornutzungsinformationen mindestens eines von einer Kennung, einem Herstellungsdatum und einer Produktionscharge des Sensormoduls (110) sind,
die generierten Sensornutzungsinformationen mindestens eines von einer Nutzungsstartzeit des Sensormoduls (110) und einer Kennung des Senders (130) sind, und
die empfangenden Sensornutzungsinformationen Benutzerinformationen sind, die von dem Benutzerendgerät (10) empfangen werden.

4. Vorrichtung zur kontinuierlichen Messung biometrischer Informationen nach Anspruch 3, wobei das Sensormodul (110) ferner eine Speichersteuerung (113) beinhaltet, die dazu konfiguriert ist, die generierten Sensornutzungsinformationen zu generieren, die generierten Sensornutzungsinformationen und in dem Speicher (115) zu registrieren und zu speichern, die empfangenen Sensornutzungsinformationen zu empfangen und die empfangenen Sensornutzungsinformationen in dem Speicher (115) zu registrieren und zu speichern.

5. Vorrichtung zur kontinuierlichen Messung biometrischer Informationen nach Anspruch 4, wobei die Speichersteuerung (113) dazu konfiguriert ist, eine Verbindungszeit oder eine anfängliche Paarungszeit als Informationen über die Nutzungsstartzeit zu generieren, indem der Verbindungszeitpunkt festgestellt wird, zu dem das Sensormodul (110) und der Sender (130) verbunden wurden, oder ein Zeitpunkt festgestellt wird, zu dem eine erste Paarungsverbindung zwischen dem Sender (130) und dem Benutzerendgerät (10) hergestellt wurde, und die generierten Nutzungsstartinformationen in dem Speicher (115) zu registrieren und zu speichern.

6. Vorrichtung zur kontinuierlichen Messung biometrischer Informationen nach Anspruch 4, wobei das Sensormodul (110) ferner eine Betriebssteuerung (111) umfasst, die dazu konfiguriert ist, auf der Grundlage der registrierten und gespeicherten Senderkennung festzustellen, ob ein neuer Sender mit dem Sensormodul verbunden ist, und wenn der neue Sender mit dem Sensormodul verbunden ist, kontinuierliches Messen der biometrischen Informationen, ohne einen Initialisierungsvorgang des Sensormoduls durchzuführen.

7. Vorrichtung zur kontinuierlichen Messung biometrischer Informationen nach Anspruch 4, wobei das Sensormodul (110) ferner einen Nachrichtenmanager (119) umfasst, der dazu konfiguriert ist, aus einer Kombination der gemessenen biometrischen Informationen und der registrierten und gespeicherten Benutzerinformationen eine Messnachricht zu generieren und die Messnachricht über den Sender (130) an das Benutzerendgerät (10) zu senden.

8. Vorrichtung zur kontinuierlichen Messung biometrischer Informationen nach Anspruch 7, wobei der Nachrichtenmanager (119) dazu konfiguriert ist, die Messnachricht durch Verschlüsselung der gemessenen biometrischen Informationen mit den Benutzerinformationen zu generieren.

9. Vorrichtung zur kontinuierlichen Messung biometrischer Informationen nach Anspruch 4, wobei die Speichersteuerung (113) beim Generieren der Nutzungsstartzeitinformationen dazu konfiguriert ist, registrierte und gespeicherte Herstellungsdatumsinformationen oder Produktionschargeninformationen des Sensormoduls (110) an das Benutzerendgerät (10) über den Sender (130) zu senden,
wobei die Herstellungsdatumsinformationen von dem Benutzerendgerät (10) verwendet werden, um den Ablaufzeitraum des Sensormoduls (110) festzustellen, oder Produktionschargeninformationen von dem Benutzerendgerät (10) verwendet werden, um festzustellen, ob das Sensormodul (110) defekt ist.

10. Vorrichtung zur kontinuierlichen Messung biometrischer Informationen nach Anspruch 7, wobei der Nachrichtenmanager (119) dazu konfiguriert ist, die Nutzungsdauer des Sensormoduls (110) aus den Nutzungsstartzeitinformationen festzustellen und, wenn festgestellt wird, dass die Nutzungsdauer des Sensormoduls abgelaufen ist, eine Ablaufnachricht zu generieren und die Ablaufnachricht über den Sender (130) an das Benutzerendgerät (10) zu senden.

## Revendications

1. Dispositif de mesure continue d'informations biométriques comprenant :
un module de capteur (110) placé sur la peau d'un utilisateur et mesurant périodiquement des informations biologiques de l'utilisateur à partir du fluide corporel, le module de capteur (110) comprenant une mémoire distincte (115) destinée à stocker des informations d'utilisation de capteur en son sein ; et
un émetteur (130) connecté au module de capteur (110) de manière amovible pour transmettre les informations biométriques mesurées par le module de capteur à un terminal utilisateur (10),
**caractérisé en ce que :**
le module de capteur (110) est en outre configuré pour : recevoir un identifiant d'émetteur de la part de l'émetteur (130) lors de la connexion de l'émetteur au module de capteur et stocker l'identifiant transmis dans la mémoire (115) ;
déterminer si l'émetteur est un émetteur remplacé ;
en réponse à la détermination selon laquelle l'émetteur est un émetteur remplacé, comparer l'identifiant d'émetteur stocké à un identifiant d'émetteur précédemment stocké pour déterminer si l'émetteur remplacé est un nouvel émetteur ; et
si l'émetteur remplacé est déterminé comme étant un nouvel émetteur, mesurer en continu les informations biométriques sans effectuer une opération d'initialisation du module de capteur (110).

2. Dispositif de mesure continue d'informations biométriques selon la revendication 1, lesdites informations d'utilisation de capteur comprenant des informations d'utilisation de capteur enregistrées qui sont précédemment enregistrées dans la mémoire (115), des informations d'utilisation de capteur générées qui sont générées lorsque le module de capteur (110) et l'émetteur (130) sont connectés, et des informations d'utilisation de capteur reçues qui sont reçues du terminal utilisateur (10) par l'intermédiaire de l'émetteur (130).

3. Dispositif de mesure continue d'informations biométriques selon la revendication 2, lesdites informations d'utilisation de capteur enregistrées étant au moins un parmi un identifiant, une date de fabrication et un lot de production du module de capteur (110),
lesdites informations d'utilisation de capteur générées étant au moins un parmi une heure de début d'utilisation du module de capteur (110) et un identifiant de l'émetteur (130), et
lesdites informations d'utilisation de capteur de réception étant des informations utilisateur reçues du terminal utilisateur (10).

4. Dispositif de mesure continue d'informations biométriques selon la revendication 3, ledit module de capteur (110) comprenant en outre un dispositif de commande de stockage (113) configuré pour générer les informations d'utilisation de capteur générées, enregistrer et stocker les informations d'utilisation de capteur générées dans la mémoire (115), recevoir les informations d'utilisation de capteur reçues, et enregistrer et stocker les informations d'utilisation de capteur reçues dans la mémoire (115).

5. Dispositif de mesure continue d'informations biométriques selon la revendication 4, ledit dispositif de commande de stockage (113) étant configuré pour générer un temps de connexion ou un temps d'appairage initial en tant qu'informations sur l'heure de début d'utilisation en déterminant le temps de connexion lorsque le module de capteur (110) et l'émetteur (130) sont connectés ou en déterminant le moment où une connexion d'appairage initiale est établie entre l'émetteur (130) et le terminal utilisateur (10) et enregistrer et stocker les informations de début d'utilisation générées dans la mémoire (115).

6. Dispositif de mesure continue d'informations biométriques selon la revendication 4, ledit module de capteur (110) comprenant en outre un dispositif de commande de fonctionnement (111) configuré pour déterminer si un nouvel émetteur est connecté au module de capteur sur la base de l'identifiant d'émetteur enregistré et stocké, et si le nouvel émetteur est connecté au module de capteur, mesurer en continu les informations biométriques sans effectuer une opération d'initialisation du module de capteur.

7. Dispositif de mesure continue d'informations biométriques selon la revendication 4, ledit module de capteur (110) comprenant en outre un gestionnaire de messages (119) configuré pour générer un message de mesure à partir d'une combinaison des informations biométriques mesurées et des informations utilisateur enregistrées et stockées et transmettre le message de mesure au terminal utilisateur (10) par l'intermédiaire de l'émetteur (130).

8. Dispositif de mesure continue d'informations biométriques selon la revendication 7, ledit gestionnaire de messages (119) étant configuré pour générer le message de mesure en cryptant les informations biométriques mesurées avec les informations utilisateur.

9. Dispositif de mesure continue d'informations biométriques selon la revendication 4, lors de la génération des informations d'heure de début d'utilisation, ledit dispositif de commande de stockage (113) étant configuré pour transmettre des informations de date de fabrication enregistrées et stockées ou des informations de lot de production du module de capteur (110) au terminal utilisateur (10) par l'intermédiaire de l'émetteur (130),
lesdites informations de date de fabrication étant utilisées par le terminal utilisateur (10) pour déterminer la période d'expiration du module de capteur (110), ou les informations de lot de production étant utilisées par le terminal utilisateur (10) pour déterminer si le module de capteur (110) est défectueux.

10. Dispositif de mesure continue d'informations biométriques selon la revendication 7, ledit gestionnaire de messages (119) étant configuré pour déterminer la période d'utilisation du module de capteur (110) à partir des informations d'heure de début d'utilisation et, lorsque la période d'utilisation du module de capteur est déterminée comme étant expirée, générer un message d'expiration et transmettre le message d'expiration au terminal utilisateur (10) par l'intermédiaire de l'émetteur (130).
